# EUROPEAN PATENT APPLICATION

(11) **EP 3 866 175 A2**
(43) Date of publication of application: **18.08.2021**
(21) Application number: 19885108.1
(22) Date of filing: 18.11.2019
(51) Int. Cl.: G16H 50/20

(54) **SUPERVISED LEARNING-BASED CONSENSUS DIAGNOSIS METHOD AND SYSTEM THEREOF**

(30) Priority: 16.11.2018 KR 20180142095
(71) Applicant: Deep Bio Inc., Seoul 08394 (KR)
(72) Inventor: KIM, Sun Woo, Seongnam-si Gyeonggi-do 13599 (KR)
(74) Representative: Flügel Preissner Schober Seidel
(86) International application number: PCT/KR2019/015737
(87) International publication number: WO 2020/101457

(57) **Abstract**

Disclosed are a supervised learning-based consensus diagnosis method and a system thereof. The supervised learning-based consensus diagnosis method comprises: a step of confirming, by a consensus diagnostic system, N individual diagnosis results in which each of N (N is an integer of 2 or more) diagnostic systems receives and outputs predetermined biological data, wherein the N diagnostic systems, respectively, are systems that are each trained with learning data annotated by different annotation subjects; and a step of outputting a consensus diagnosis result of the biological data on the basis of the individual diagnosis results confirmed by the consensus diagnosis system.

## Description

### [Technical Field]

The present disclosure relates to a method and system for diagnosing a disease through learning, and more particularly, to a method and system, which includes a plurality of supervised learning-based diagnosis systems trained through neural networks and can supplement a weak point of learning based on supervised learning by deriving the final diagnosis result through a consensus among the plurality of diagnosis systems.

### [Background Art]

One of major tasks performed in pathology or a pathology department is a task for performing a diagnosis for determining a state or symptom of a specific disease by reading a bio image of a patient. Such a diagnosis is a method dependent on experiences and knowledge of a healthcare worker experienced for a long period.

Recently, with the development of machine learning, attempts to automate a task, such as recognizing or classifying images, using a computer system are actively made. In particular, attempts to automate a diagnosis performed by an experienced healthcare worker by using a neural network (e.g., a deep learning method using a convolutional neural network (CNN)), that is, a kind of machine learning, is carried out.

In particular, a diagnosis through deep learning using a neural network (e.g., CNN) also includes a case where a characteristic of a disease feature not found by an experienced healthcare worker is found out in an image in that a desired solution is derived by autonomously finding out characteristic elements through learning without simply automating experiences and knowledge of an experienced healthcare worker as in a conventional technology.

In general, a diagnosis of a disease through a neural network using bio data (e.g., bio image) includes that an experienced healthcare worker annotates the bio data with a state of a specific disease (e.g., whether cancer has developed). The neural network is trained using such multiple annotated data as learning data. That is, learning data is annotated for learning, and learning through the annotated learning data is chiefly used. Such a learning method is called supervised learning.

However, in the supervised learning, performance of a trained system is dominantly influenced by a decision propensity or tendency of an annotator who has performed annotation. That is, a diagnosis result of a trained diagnosis system is dependent on an annotation tendency or propensity of an annotator.

However, bio data to be learned actually includes a large amount of bio data that is difficult to be clearly classified, to the extent that when actually performing annotation, annotators perform different annotation. That is, although an experienced healthcare worker performs annotation, he or she may have a different opinion on whether a disease has developed or classifying a degree of the progress of the disease depending on bio data.

Nevertheless, a disease diagnosis system trained based on supervised learning has a weak point in that a determination dependent on an annotator's tendency is output as a diagnosis result.

Accordingly, there is a need for a technical spirit capable of improving such a weak point.

### *Prior Art Document

### -Patent Document

Korean Patent No. 10-20170057399 "SYSTEM FOR DIAGNOSING DISEASE THROUGH MODULARIZED REINFORCEMENT LEARNING"

### [Disclosure]

### [Technical Problem]

An object of the present disclosure is to provide a method and system, which includes a plurality of supervised learning-based diagnosis systems trained by different annotation subjects and can supplement a weak point based on supervised learning by deriving the final diagnosis result, that is, a diagnosis result agreed among the diagnosis systems, based on diagnosis results of given bio data, which are performed by the plurality of diagnosis systems.

Furthermore, an object of the present disclosure is to provide a method and system which can also improve performance of individual diagnosis systems in such a manner that the individual diagnosis systems are retrained using diagnosis results based on a consensus among the diagnosis systems.

### [Technical Solution]

A method of diagnosing a disease by using a diagnosis system trained through supervised learning for achieving the technical object includes the steps of checking, by a consensus diagnosis system, N individual diagnosis results of given bio data, which are output by respective N (N is an integer equal to or greater than 2) diagnosis systems, wherein each of the N diagnosis systems is a system trained using learning data annotated by a different annotation subject; and outputting, by the consensus diagnosis system, a consensus diagnosis result of the bio data based on the checked individual diagnosis results.

The supervised learning-based consensus diagnosis method further includes the step of checking a standard-individual diagnosis result of the bio data, which is output by a standard diagnosis system trained using gold standard learning data. The step of outputting, by the consensus diagnosis system, the consensus diagnosis result of the bio data based on the checked individual diagnosis results may include the step of outputting the consensus diagnosis result based on the individual diagnosis results and the standard-individual diagnosis result.

The step of outputting, by the consensus diagnosis system, the consensus diagnosis result of the bio data based on the checked individual diagnosis results includes outputting the consensus diagnosis result based on a weight assigned to each of the diagnosis systems. The weight may be determined based on a number of times that a specific diagnosis system outputs an individual diagnosis result different from the consensus diagnosis result.

The weight may be determined further based on the number or percentage of other diagnosis systems which output individual diagnosis results different from the consensus diagnosis result among all the individual diagnosis results, when the specific diagnosis system outputs the individual diagnosis result different from the consensus diagnosis result.

The step of outputting, by the consensus diagnosis system, the consensus diagnosis result of the bio data based on the checked individual diagnosis results includes outputting the consensus diagnosis result based on a weight assigned to each of the diagnosis systems and the standard diagnosis system. A higher weight may be assigned to the standard diagnosis system than to the diagnosis systems.

The diagnosis systems are systems which learn N split learning data sets split from a given learning data set and output diagnosis results of a disease, respectively. A given split learning data set may include at least one learning data not included in other split learning data sets, and the N split learning data sets are learning data sets annotated by different subjects, respectively.

The supervised learning-based consensus diagnosis method may further include the step of retraining a diagnosis system, having an individual diagnosis result of specific bio data different from a consensus diagnosis result of the specific bio data, by using learning data including the specific bio data annotated as the consensus diagnosis result.

The method may be implemented by a computer program installed in a data processing apparatus and stored in a computer-readable recording medium.

A system for achieving the technical object includes a processor and a storage device in which a program is stored. The program checks N individual diagnosis results of given bio data, which are output by respective N (N is an integer equal to or greater than 2) diagnosis systems, wherein each of the N diagnosis systems is a system trained using learning data annotated by a different annotation subject, and outputs a consensus diagnosis result of the bio data based on the checked individual diagnosis results.

The program may further check a standard-individual diagnosis result of the bio data, which is output by a standard diagnosis system trained by gold standard learning data, and may output the consensus diagnosis result based on the individual diagnosis results and the standard-individual diagnosis result.

The program may output the consensus diagnosis result based on a weight assigned to each of the diagnosis systems, and the weight may be determined based on a number of times that the consensus diagnosis result and each of individual diagnosis results of the diagnosis systems are different.

The weight may be determined further based on a number or percentage of other diagnosis systems which output individual diagnosis results different from the consensus diagnosis result among all the individual diagnosis results, when the specific diagnosis system outputs the individual diagnosis result different from the consensus diagnosis result.

The program may output the consensus diagnosis result based on a weight assigned to each of the diagnosis systems and the standard diagnosis system, and a higher weight may be assigned to the standard diagnosis system than to the diagnosis systems.

Furthermore, the consensus diagnosis result and an individual diagnosis result of a specific diagnosis system among the diagnosis systems may be different with respect to specific bio data of the bio data used for the diagnosis of the consensus diagnosis system, and the specific diagnosis system may be retrained by learning data including the specific bio data annotated as the consensus diagnosis result.

### [Advantageous Effects]

According to the technical spirit of the present disclosure, there is an effect in that the dependence of a diagnosis result on annotation, which may occur when a disease is diagnosed through a supervised learning-based diagnosis system trained using learning data annotated by any one annotation subject, can be supplemented.

Furthermore, individual diagnosis systems can be retrained using a diagnosis result through a consensus according to the technical spirit of the present disclosure. In such a case, there is an effect in that performance of the individual diagnosis systems can also be improved.

### [Description of Drawings]

A brief description of the drawings is provided so that the drawings cited in the detailed description of the present disclosure are sufficiently understood.
FIG. 1 is a diagram illustrating a schematic system configuration for implementing a supervised learning-based consensus diagnosis method according to the technical spirit of the present disclosure.
FIG. 2 is a diagram for describing a schematic configuration of each of diagnosis systems according to an embodiment of the present disclosure.
FIG. 3 is a diagram for describing the concept that a consensus based on individual diagnosis results is derived according to an embodiment of the present disclosure.
FIG. 4 is a diagram for describing a split annotation concept of learning data according to an embodiment of the present disclosure.
FIG. 5 is a diagram for describing a concept that individual diagnosis systems are retrained using a consensus diagnosis result according to an embodiment of the present disclosure.

### [Best Mode for Invention]

The present disclosure may be variously changed and may have various embodiments. Specific embodiments will be illustrated in the drawings and will be described in detail in the detailed description. It is however to be understood that the present disclosure is not intended to be limited to the specific embodiments and includes all changes, equivalents and substitutions included in the spirit and technical range of the present disclosure. In describing the present disclosure, a detailed description of a related known technology will be omitted if it is deemed to make the gist of the present disclosure unnecessarily vague.

Terms, such as "first" and "second", may be used to describe various elements, but the elements should not be restricted by the terms. The terms are used to only distinguish one element from the other element.

The terms used in this application are used to merely describe specific embodiments and are not intended to restrict the present disclosure. An expression of the singular number includes an expression of the plural number unless clearly defined otherwise in the context.

In this specification, it is to be understood that a term, such as "include" or "have", is intended to designate that a characteristic, a number, a step, an operation, an element, a part or a combination of them described in the specification is present, and does not exclude the presence or addition possibility of one or more other characteristics, numbers, steps, operations, elements, parts, or combinations of them in advance.

Furthermore, in this specification, if any one element "transmits" data to another element, this means that the one element may directly transmit the data to the another element or may transmit the data to the another element through at least another element. In contrast, if any one element "directly transmits" data to another element, this means that the data is transmitted the another element without the intervention of another element.

Hereinafter, the present disclosure is described in detail based on embodiments of the present disclosure with reference to the accompanying drawings. The same reference numerals described in drawings refer to the same elements.

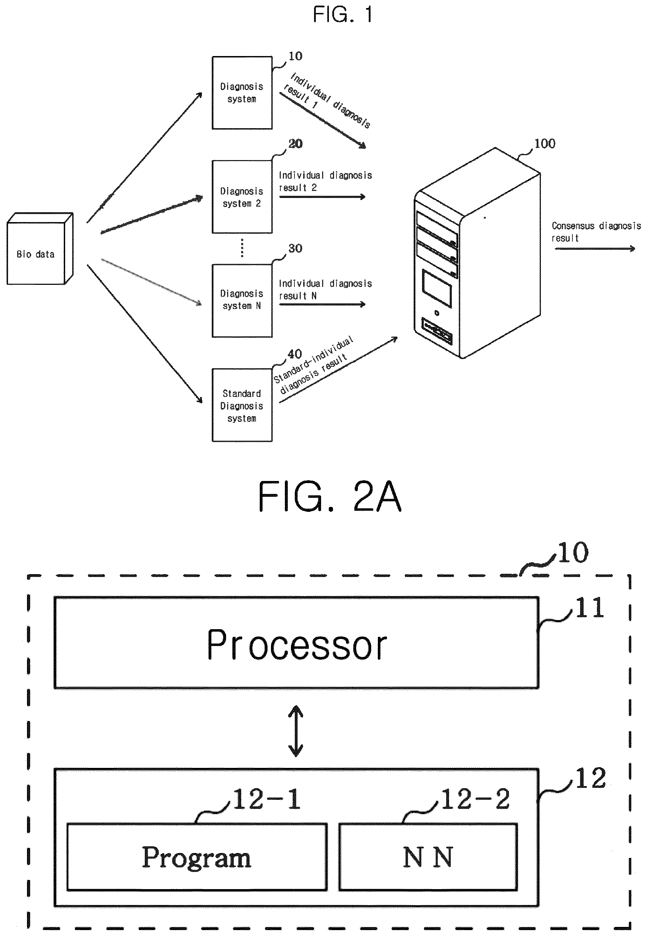

FIG. 1 is a diagram illustrating a schematic system configuration for implementing a supervised learning-based consensus diagnosis method according to the technical spirit of the present disclosure.

Referring to FIG. 1, in order to implement the supervised learning-based consensus diagnosis method according to the technical spirit of the present disclosure, a consensus diagnosis system 100 may be implemented.

The consensus diagnosis system 100 may receive individual diagnosis results (e.g., an individual diagnosis result 1 to an individual diagnosis result N) from a plurality (e.g., N, N is an integer equal to or greater than 2) of individual diagnosis systems (e.g., a diagnosis system 1 to a diagnosis system N, 10 to 30) according to the technical spirit of the present disclosure. Furthermore, the consensus diagnosis system 100 may output a consensus diagnosis result based on the received individual diagnosis results (e.g., the individual diagnosis result 1 to the individual diagnosis result N).

Each of diagnosis systems (e.g., the diagnosis system 1 to the diagnosis system N, 10 to 30) may be a system, which is trained based on supervised learning, classifies given bio data (e.g., bio image) into predetermined classes (e.g., classes according to whether a disease has developed or a progress degree of the disease) through a trained neural network when receiving the bio data, and outputs a corresponding result as a diagnosis result.

Diagnosis systems (e.g., the diagnosis system 1 to the diagnosis system N, 10 to 30) may receive the same bio data and output diagnosis results, that is, individual diagnosis results (e.g., the individual diagnosis result 1 to the individual diagnosis result N), through respective trained neural networks.

As described above, a diagnosis result of each of diagnosis systems (e.g., the diagnosis system 1 to the diagnosis system N, 10 to 30) may be dependent on annotation according to an annotator. That is, each of the diagnosis systems (e.g., the diagnosis system 1 to the diagnosis system N, 10 to 30) may output the individual diagnosis result in a way to have dependence on annotated learning data.

In order to supplement such a weak point, each of diagnosis systems (e.g., the diagnosis system 1 to the diagnosis system N, 10 to 30) according to the technical spirit of the present disclosure may be a system trained by a learning data set annotated by a different annotation subject.

For example, the diagnosis system 1 10 may be a system trained by a first learning data set annotated by a first annotator. The diagnosis system 2 20 may be a system trained by a second learning data set annotated by a second annotator. The diagnosis system N 30 may be a system trained by an N-th learning data set annotated by an N-th annotator.

In such a case, each of diagnosis systems (e.g., the diagnosis system 1 to the diagnosis system N, 10 to 30) may have a characteristic dependent on the annotator's determination propensity or tendency to the learning data used for the training.

For example, it is preferred that diagnosis systems (e.g., the diagnosis system 1 to the diagnosis system N, 10 to 30) that have received the same bio data (e.g., bio image) ideally output the same diagnosis results. However, as described above, the diagnosis systems may output different diagnosis results in some cases because the annotator's determination propensity affects the diagnosis result. That is, the diagnosis systems (e.g., the diagnosis system 1 to the diagnosis system N, 10 to 30) may output different diagnosis results of the same bio data depending on the annotators' propensities.

However, such a weak point may be supplemented through a consensus diagnosis using individual diagnosis results of a plurality of diagnosis systems (e.g., the diagnosis system 1 to the diagnosis system N, 10 to 30) according to the technical spirit of the present disclosure.

The consensus diagnosis system 100 may output a consensus diagnosis result based on individual diagnosis results output by diagnosis systems (e.g., the diagnosis system 1 to the diagnosis system N, 10 to 30), respectively. The consensus diagnosis result may mean the final diagnosis result generated based on some of or all the individual diagnosis results.

In order to output the consensus diagnosis result, if the individual diagnosis results have different diagnosis results, the consensus diagnosis system 100 may simply derive the consensus diagnosis result based on a majority vote. Alternatively, the consensus diagnosis system 100 may assign weights to the diagnosis systems (e.g., the diagnosis system 1 to the diagnosis system N, 10 to 30), respectively, and may output a consensus diagnosis result applied to the individual diagnosis results corresponding to the assigned weights.

The weight may be assigned based on the diagnosis history of each of the diagnosis systems (e.g., the diagnosis system 1 to the diagnosis system N, 10 to 30). For example, a weight assignment process may be driven by the consensus diagnosis system 100 so that a weight assigned to a diagnosis system that outputs a diagnosis result different from a consensus diagnosis result is decreased by a given reference. Furthermore, in the weight assignment process, only whether a diagnosis result is different from a consensus diagnosis result may be considered, but the number of diagnosis results for each type among all individual diagnosis results may be further considered.

For example, if a consensus diagnosis is implemented by implementing ten diagnosis systems, any one specific diagnosis system of the ten diagnosis systems may determine that a disease has not developed, and all the remaining diagnosis systems may determine that the disease has developed, with respect to specific bio data. In such a case, a weight for the specific diagnosis system may be decreased by a predetermined first reference value (e.g., 0.1).

However, four diagnosis systems of the ten diagnosis systems may determine that the disease has not developed with respect to the specific bio data, and all of the six diagnosis systems may determine that the disease has developed with respect to the specific bio data. In such a case, although a consensus diagnosis result indicates that the disease has developed, weights for the four diagnosis systems may be decreased by only a given second reference value (e.g., 0.05) smaller than the first reference value (e.g., 0.1).

That is, assuming that the consensus diagnosis result is a correct answer, a weight for a diagnosis system may be simply adjusted based on the number of wrong answers output by the diagnosis system. However, although a specific diagnosis system outputs a wrong answer, a degree that the weight is adjusted may be changed by further considering the number of diagnosis systems that have output wrong answers or a weight may not be adjusted by determining that an answer is not a wrong answer according to circumstances.

In addition, an average expert of a field to which the technology of the present disclosure pertains may easily infer that various embodiments may be possible for a method of deriving a consensus diagnosis result and methods of assigning a weight to each of the diagnosis systems (e.g., the diagnosis system 1 to the diagnosis system N, 10 to 30).

In an embodiment, the consensus diagnosis system 100 may further receive a standard-individual diagnosis result from a standard diagnosis system 40 which will be described later. Furthermore, the consensus diagnosis system 100 may output a consensus diagnosis result further based on the received standard-individual diagnosis result.

The standard diagnosis system 40 may mean a diagnosis system trained by gold standard learning data. The gold standard learning data may mean learning data on which standard or ideal annotation has been performed. Such gold standard learning data may mean data that has not been simply annotated by any one subject, but on which annotation agreed by a plurality of subjects has been performed or on which annotation has been performed based on a criterion agreed by multiple subjects. In general, it may be preferred that learning is performed using such gold standard learning data. However, practically, to generate the gold standard learning data may require lots of costs. To construct a consensus diagnosis result by using a plurality of diagnosis systems trained by individual subjects rather than constructing one diagnosis system trained using gold standard learning data may have better diagnosis performance. The reason for this is that in the case of the gold standard learning data, if annotation opinions on annotation for specific bio data are different, a minority opinion is neglected and only agreed annotation is incorporated into learning, but if diagnosis systems (e.g., the diagnosis system 1 to the diagnosis system N, 10 to 30) individually trained through annotation of individual subjects are constructed as in the technical spirit of the present disclosure, a minority opinion is at least incorporated into an individual diagnosis system for training.

Moreover, if the standard diagnosis system 40 is included in a plurality of diagnosis systems for a consensus diagnosis, higher diagnosis performance may be obtained.

Furthermore, the consensus diagnosis system 100 may derive a consensus result by assigning a higher weight to the standard diagnosis system 40 than to each of individual diagnosis systems (e.g., the diagnosis system 1 to the diagnosis system N, 10 to 30) and incorporating the weight assigned to each of the diagnosis systems (e.g., the diagnosis system 1 to the diagnosis system N, 10 to 30).

Meanwhile, a case where the consensus diagnosis system 100 is implemented as a separate physical device different from diagnosis systems (e.g., the diagnosis system 1 to the diagnosis system N, 10 to 30) or the standard diagnosis system 40 is illustrated in FIG. 1, but an ordinary skilled person of a field to which the technology of the present disclosure pertains may easily infer that the consensus diagnosis system 100 may be implemented in an apparatus physically identical with at least one of diagnosis systems (e.g., the diagnosis system 1 to the diagnosis system N, 10 to 30) or the standard diagnosis system 40.

The consensus diagnosis system 100 may be implemented using various data processing systems (e.g., a computer, a server, a smartphone or a dedicated device) if the consensus diagnosis system can have only to perform a function defined in this specification.

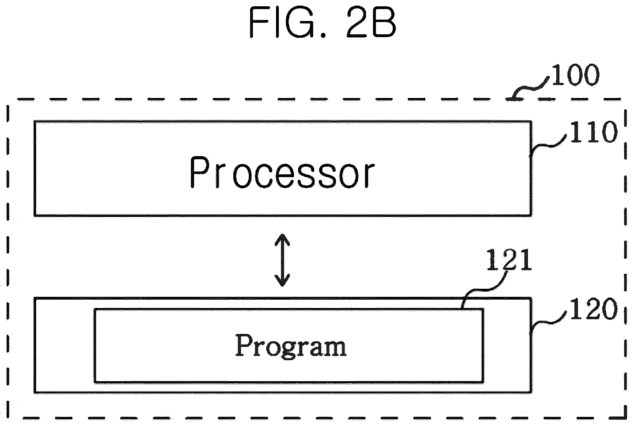

FIG. 2 is a diagram for describing a schematic configuration of each of diagnosis systems according to an embodiment of the present disclosure.

First, referring to FIG. 2a, individual diagnosis systems (e.g., the diagnosis systems (e.g., the diagnosis system 1 to the diagnosis system N, 10 to 30)) may include elements, such as those illustrated in FIG. 2a. The standard diagnosis system 40 may be implemented using the same elements as each of the individual diagnosis systems (e.g., the diagnosis system 1 to the diagnosis system N, 10 to 30). Accordingly, in this specification, only the individual diagnosis systems (e.g., the diagnosis system 1 to the diagnosis system N, 10 to 30) are described. Furthermore, the individual diagnosis systems (e.g., the diagnosis system 1 to the diagnosis system N, 10 to 30) may include the same or similar elements. Accordingly, in this specification, only the elements of any one of the diagnosis systems (e.g., the diagnosis system 1 to the diagnosis system N, 10 to 30) will be described.

The first diagnosis system 10 may include a processor 11 and a storage device 12. The first diagnosis system 10 means a data processing apparatus having the computation ability for implementing the technical spirit of the present disclosure. In general, an ordinary skilled person of a field to which the technology of the present disclosure pertains may easily infer that the first diagnosis system may be implemented as any device capable of performing a specific service, such as a personal computer or a portable terminal, in addition to a data processing apparatus accessible to a client over a network.

The processor 11 may mean a computation device capable of driving a program 12-1 for implementing the technical spirit of the present disclosure. The processor 11 may performs a diagnosis by using the program 12-1 and a neural network 12-2 defined by the technical spirit of the present disclosure. The neural network may be a convolutional neural network, and may output a diagnosis result through the neural network when receiving bio data (e.g., an image).

The program 12-1 may mean software that can train the neural network 12-2 through supervised learning or perform a diagnosis by using the trained neural network 12-2.

The storage device 12 may mean data storage means capable of storing the program 12-1 and the neural network 12-2. According to an implementation example, the storage device may be implemented as a plurality of storage means. Furthermore, the storage device 12 may be a meaning including a temporary storage device or a memory which may be included in the processor 11, in addition to a main storage device included in the first diagnosis system 10.

The diagnosis system 100 has been illustrated as being implemented as any one physical device in FIG. 2. However, an ordinary skilled person of a field to which the technology of the present disclosure pertains may easily infer that a plurality of physical devices may be organically combined to implement the first diagnosis system 10 according to the technical spirit of the present disclosure, if necessary.

Hereinafter, in this specification, when it is said that a diagnosis system (e.g., 10) performs a given function, this may mean that a processor (e.g., 11) included in the diagnosis system (e.g., 10) performs the given function by using a program (e.g., 12-1).

In this specification, when it is said that the first diagnosis system 10 performs a diagnosis, this may mean a series of processes of receiving bio data and outputting output data defined in this specification, for example, a diagnosis result.

The first diagnosis system 10 may receive bio data in unit of a given unit. The given unit may be a pixel, a patch, or a slide, for example.

A diagnosis result of the first diagnosis system 10 may be simply whether a disease has developed or a corresponding value (e.g., a probability) depending on the type of disease or may be state information indicating a degree of state of a disease if the disease has developed.

For example, as will be described later, if the technical spirit of the present disclosure is used to diagnose prostate cancer, a Gleason pattern or a Gleason score, that is, an index indicative of a degree of the progress of prostate cancer, may be included in the state information. For example, the Gleason pattern has a value from 2 to 5. A higher value indicates that a degree that prostate cancer has developed is severe. Accordingly, the state information may include information corresponding to the probability that a biological tissue corresponding to the unit of a unit, that is, a target of diagnosis, may correspond to a specific value (e.g., 3, 4, or 5) or the Gleason pattern or information corresponding to the probability that the biological tissue may correspond to "normal" (i.e., if a disease has not developed).

In any case, the first diagnosis system 10 may output a diagnosis result through the trained neural network 12-2.

Meanwhile, the consensus diagnosis system 100 according to the technical spirit of the present disclosure may include elements illustrated in FIG. 2b.

The consensus diagnosis system 100 may also include a processor 110 and a storage device 120 in which a program 121 is stored.

The consensus diagnosis system 100 is connected to individual diagnosis systems (e.g., the diagnosis system 1 to the diagnosis system N, 10 to 30) and/or the standard diagnosis system 40 over a wired/wireless network, and may transmit and receive information necessary to implement the technical spirit of the present disclosure. According to an implementation example, the consensus diagnosis system 100 may be implemented by being installing in the individual diagnosis systems (e.g., the diagnosis system 1 to the diagnosis system N, 10 to 30) or the standard diagnosis system 40. In such a case, the consensus diagnosis system 100 may share the hardware of the individual diagnosis systems (e.g., the diagnosis system 1 to the diagnosis system N, 10 to 30) or the standard diagnosis system 40.

The program 121 stored in the storage device 120 of the consensus diagnosis system 100 may receive and check diagnosis results (e.g., the individual diagnosis result 1 to N and/or a standard-individual diagnosis result) from individual diagnosis systems (e.g., the diagnosis system 1 to the diagnosis system N, 10 to 30) and/or the standard diagnosis system 40, respectively.

The consensus diagnosis system 100 may receive individual diagnosis results from only diagnosis systems (e.g., the diagnosis system 1 to the diagnosis system N, 10 to 30) trained by learning data sets annotated by different annotation subjects, respectively, but may further receive a standard-individual diagnosis result from the standard diagnosis system 40 as described above.

In any case, the consensus diagnosis system 100 may generate and output a consensus diagnosis result based on the received diagnosis results.

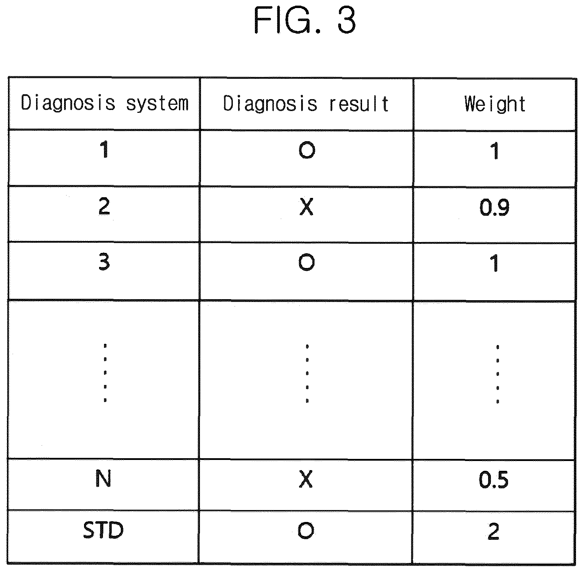

An example in which the consensus diagnosis system 100 generates the consensus diagnosis result is described with reference to FIG. 3.

FIG. 3 is a diagram for describing the concept that a consensus based on individual diagnosis results is derived according to an embodiment of the present disclosure.

Referring to FIG. 3, in order to implement the technical spirit of the present disclosure, diagnosis systems (e.g., the diagnosis system 1 to the diagnosis system N, 10 to 30) may be implemented. The consensus diagnosis system 100 may receive diagnosis results of the same bio data from the diagnosis systems (e.g., the diagnosis system 1 to the diagnosis system N, 10 to 30), respectively. Furthermore, FIG. 3 illustrates a case where the consensus diagnosis system 100 further receives a standard-individual diagnosis result from the standard diagnosis system (STD) 40.

FIG. 3 illustrates a case where the diagnosis result indicates whether a specific disease has developed. However, as described above, various types of information may be output as diagnosis results depending on an implementation example of the neural network 12-2.

According to an example, the consensus diagnosis system 100 may simply specify, as a consensus diagnosis result, a type having a greater number among types of diagnosis results (e.g., O indicating that a disease has developed and X indicating that a disease has not developed).

According to an implementation example, the consensus diagnosis system 100 may assign a weight to each of diagnosis systems (e.g., the diagnosis system 1 to the diagnosis system N, 10 to 30, the standard diagnosis system 40).

Such a weight may be adjusted based on the number of times that an individual diagnosis result different from a consensus diagnosis result has been output while the consensus diagnosis result is derived with respect to multiple bio data. For example, the diagnosis system 2 may have a weight of 0.9, and the diagnosis system 1 may have a weight of 1. This may mean that a case where the diagnosis system 2 has output an individual diagnosis result different from a consensus diagnosis result was at least one.

The consensus diagnosis system 100 may generate a consensus diagnosis result based on a diagnosis result into which a weight has been incorporated as a weight factor. For example, if a specific diagnosis system has output a diagnosis result X and a weight for the specific diagnosis system is "a" (e.g., 0.9), the diagnosis result of the specific diagnosis system may be determined to be "a" (0.9) Xs in number in a process of deriving a consensus diagnosis result. As described above, larger types of diagnosis results (e.g., O or X) may be determined as a consensus diagnosis result by incorporating weights.

The consensus diagnosis system 100 may simply adjust the weight by lowering a predetermined value (e.g., 0.1) every case where an individual diagnosis result different from a consensus diagnosis result is output for each of diagnosis systems (e.g., the diagnosis system 1 to the diagnosis system N, 10 to 30 and/or the standard diagnosis system 40).

Alternatively, as described above, although a specific diagnosis system (e.g., the diagnosis system 2) has output an individual diagnosis result (e.g., X) different from a consensus diagnosis result (e.g., O), the weight may be adjusted by further considering the number or percentage of diagnosis systems (e.g., the diagnosis system 1 to the diagnosis system N, 10 to 30 and/or the standard diagnosis system 40) that have output the different individual diagnosis result (e.g., X) (the number of diagnosis systems that have output the same type of diagnosis result as the specific diagnosis system (e.g., the diagnosis system 2) compared to the number of all diagnosis systems). For example, if only one of ten diagnosis system has output a different diagnosis result, the weight of the corresponding system may be adjusted by a first value (e.g., 0.1). If a specific number (e.g., 3) or more or a specific percentage (e.g., 30%) or more of the ten diagnosis system have output different diagnosis results, the weight of each of the corresponding systems may be adjusted by a second value (e.g., 0.03). Alternatively, in the case of a specific number or more or a specific percentage or more, although diagnosis results different from a consensus diagnosis result are output, the weights of corresponding systems may not be adjusted.

That is, in the latter case, the diagnosis results may be interpreted as being practically different diagnosis results depending on a determination. Accordingly, a further reliable diagnosis result may be generally derived by adaptively adjusting a weight corresponding to reliability of a corresponding diagnosis system.

Furthermore, if a diagnosis result of the standard diagnosis system 40 is further used, the consensus diagnosis system 100 may assign a higher weight to the standard diagnosis system 40 than to other diagnosis systems (e.g., the diagnosis system 1 to the diagnosis system N, 10 to 30).

Meanwhile, in the case of a consensus diagnosis method according to the technical spirit of the present disclosure, although diagnosis systems (e.g., the diagnosis system 1 to the diagnosis system N, 10 to 30 and/or the standard diagnosis system 40) trained by different learning data sets are used, diagnosis results can be usefully derived. As will be described later, this may be further useful for a case where the consensus diagnosis system 100 performs retraining on a diagnosis system that outputs a diagnosis result different from a consensus diagnosis result.

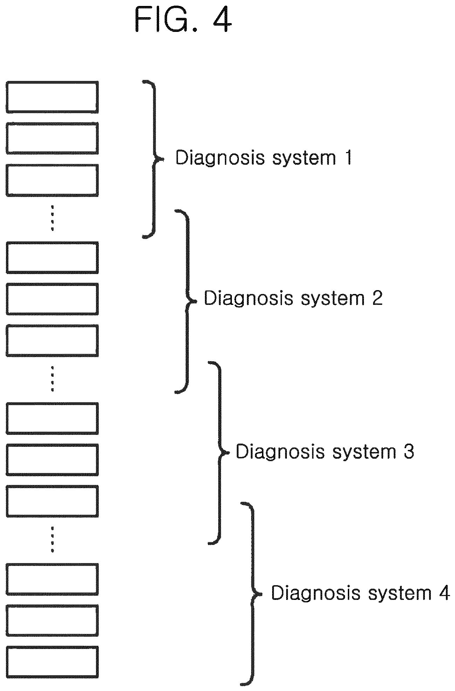

FIG. 4 is a diagram for describing a split annotation concept of learning data according to an embodiment of the present disclosure.

Referring to FIG. 4, if a consensus diagnosis method according to the technical spirit of the present disclosure is applied, as described above, the consensus diagnosis method may be useful for a case where diagnosis systems (e.g., the diagnosis system 1 to the diagnosis system N, 10 to 30 and/or the standard diagnosis system 40) are trained using different learning data, in addition to a case where diagnosis systems (e.g., the diagnosis system 1 to the diagnosis system N, 10 to 30 and/or the standard diagnosis system 40) are trained using learning data differently annotated by different annotators.

For example, as illustrated in FIG. 4, performance of a trained engine, that is, a diagnosis system, may be different depending on a propensity of an annotator that annotates learning data used for learning. Although the same annotator performs annotation, the performance of the diagnosis system is influenced depending on which learning data is used.

Accordingly, as illustrated in FIG. 4, a diagnosis system 1 may be a system trained by a learning data set 1, a diagnosis system 2 may be a system trained by a learning data set 2, a diagnosis system 3 may be a system trained by a learning data set 3, and a diagnosis system 4 may be a system trained by a learning data set 4.

Each learning data set may include or may not include redundant learning data. An annotator may be the same or different for each learning data set.

In either case, there may be a difference between diagnosis results of respective diagnosis systems trained for each learning data set. For this, conventionally, great efforts and costs must be consumed in order to determine wide learning data sets including all of various cases from the time when the learning data sets are selected.

However, according to the technical spirit of the present disclosure, if diagnosis systems are trained using a sufficient degree of learning data sets to some extent in order to train the diagnosis systems, even diagnosis systems (e.g., the diagnosis system 1 to the diagnosis system N, 10 to 30 and/or the standard diagnosis system 40) trained by different learning data sets can derive more accurate diagnosis results through a consensus diagnosis result.

That is, there is an effect in that an inclination of a learning data set itself can be significantly deflected in a process of deriving a consensus diagnosis result.

Moreover, specific bio data may be annotated as a consensus diagnosis result, and retraining may be performed on a diagnosis system that has output a diagnosis result different from the consensus diagnosis result with respect to the specific bio data. In such a case, an inclination of an annotator and/or an inclination of learning data can be deflected over time. Eventually, there is an effect in that performance of individual diagnosis systems (e.g., the diagnosis system 1 to the diagnosis system N, 10 to 30 and/or the standard diagnosis system 40) can also be improved.

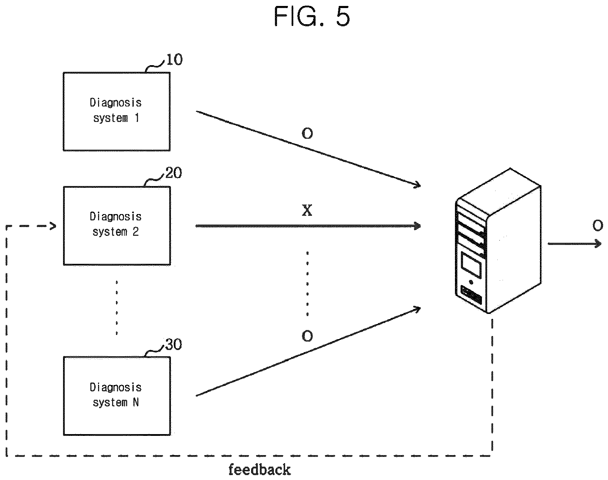

Such an example is described with reference to FIG. 5.

FIG. 5 is a diagram for describing a concept that individual diagnosis systems are retrained using a consensus diagnosis result according to an embodiment of the present disclosure.

Referring to FIG. 5, for example, specific bio data may be input to each of diagnosis systems (e.g., the diagnosis system 1 to the diagnosis system N, 10 to 30 and/or the standard diagnosis system 40). Each of the diagnosis systems (e.g., the diagnosis system 1 to the diagnosis system N, 10 to 30 and/or the standard diagnosis system 40) may output respective individual diagnosis results to the consensus diagnosis system 100. Furthermore, in this case, the consensus diagnosis system 100 may have output a consensus diagnosis result as "O", and the diagnosis system 2 20 may have output "X" as an individual diagnosis result.

In such a case, the consensus diagnosis system 100 may store information on a diagnosis system (e.g., the diagnosis system 2) that has output an individual diagnosis result different from a consensus diagnosis result, specific bio data in this case, and a consensus diagnosis result (e.g., O).

Furthermore, the diagnosis systems (e.g., the diagnosis system 1 to the diagnosis system N, 10 to 30 and/or the standard diagnosis system 40) may be retrained using the stored information.

For example, after a lapse of a given time, the diagnosis system 2 may be retrained using a re-learning data set including the specific bio data labeled as the consensus diagnosis result (e.g., O).

That is, according to the technical spirit of the present disclosure, bio data related to the output of diagnosis results different from a consensus diagnosis result may be collected, and each of diagnosis systems (e.g., the diagnosis system 1 to the diagnosis system N, 10 to 30 and/or the standard diagnosis system 40) may be retrained. That is, the bio data related to the output of the diagnosis results different from the consensus diagnosis result, stored by the consensus diagnosis system 100, may be fed back to the individual diagnosis systems as learning data. In such a case, there is an effect in that an inclination (e.g., depending on an annotator's propensity or an inclination of the learning data) of each of the diagnosis systems (e.g., the diagnosis system 1 to the diagnosis system N, 10 to 30 and/or the standard diagnosis system 40) is improved.

The consensus diagnosis method according to an embodiment of the present disclosure may be implemented in a computer-readable recording medium in the form of computer-readable code. The computer-readable recording medium includes all types of recording devices in which data readable by a computer system is stored. Examples of the recording medium may include a ROM, a RAM, a CD-ROM, a magnetic tape, a hard disk, a floppy disk, an optical data storage device. Furthermore, the computer-readable recording medium may be distributed to computer systems connected over a network, and a computer-readable code may be stored and executed in a distributed manner. Furthermore, functional programs, codes and code segments for implementing the present disclosure may be easily inferred by programmer of a technical field to which the present disclosure pertains.

Although the present disclosure has been described with reference to the embodiments illustrated in the drawings, the embodiments are only illustrative. A person having ordinary skill in the art will understand that various modifications and other equivalent embodiments are possible from the present disclosure. Accordingly, the true technical range of protection of the present disclosure should be determined by the technical spirit of the following claims.

### [Industrial Applicability]

The present disclosure may be used in a "supervised learning-based consensus diagnosis method and system."

## Claims

1. A supervised learning-based consensus diagnosis method of diagnosing a disease by using a diagnosis system trained through supervised learning, comprising steps of:
checking, by a consensus diagnosis system, N individual diagnosis results of given bio data, which are output by respective N (N is an integer equal to or greater than 2) diagnosis systems, wherein each of the N diagnosis systems is a system trained using learning data annotated by a different annotation subject; and
outputting, by the consensus diagnosis system, a consensus diagnosis result of the bio data based on the checked individual diagnosis results.

2. The supervised learning-based consensus diagnosis method of claim 1, further comprising a step of checking a standard-individual diagnosis result of the bio data, which is output by a standard diagnosis system trained using gold standard learning data,
wherein the step of outputting, by the consensus diagnosis system, the consensus diagnosis result of the bio data based on the checked individual diagnosis results comprises a step of outputting the consensus diagnosis result based on the individual diagnosis results and the standard-individual diagnosis result.

3. The supervised learning-based consensus diagnosis method of claim 1, wherein:
the step of outputting, by the consensus diagnosis system, the consensus diagnosis result of the bio data based on the checked individual diagnosis results comprises outputting the consensus diagnosis result based on a weight assigned to each of the diagnosis systems, and
the weight is determined based on a number of times that a specific diagnosis system outputs an individual diagnosis result different from the consensus diagnosis result.

4. The supervised learning-based consensus diagnosis method of claim 3, wherein the weight is determined further based on a number or percentage of other diagnosis systems which output individual diagnosis results different from the consensus diagnosis result among all the individual diagnosis results, when the specific diagnosis system outputs the individual diagnosis result different from the consensus diagnosis result.

5. The supervised learning-based consensus diagnosis method of claim 2, wherein:
the step of outputting, by the consensus diagnosis system, the consensus diagnosis result of the bio data based on the checked individual diagnosis results comprises outputting the consensus diagnosis result based on a weight assigned to each of the diagnosis systems and the standard diagnosis system, and
a higher weight is assigned to the standard diagnosis system than to the diagnosis systems.

6. The supervised learning-based consensus diagnosis method of claim 1, wherein:
the diagnosis systems are systems which learn N split learning data sets split from a given learning data set and output diagnosis results of a disease, respectively, and
a given split learning data set comprises at least one learning data not included in other split learning data sets, and the N split learning data sets are learning data sets annotated by different subjects, respectively.

7. The supervised learning-based consensus diagnosis method of claim 1, further comprising a step of retraining a diagnosis system, having an individual diagnosis result of specific bio data different from a consensus diagnosis result of the specific bio data, by using learning data comprising the specific bio data annotated as the consensus diagnosis result.

8. A computer program installed in a data processing apparatus and stored in a computer-readable recording medium for performing the method according to any one of claims 1 to 7.

9. A data processing system comprising:
a processor; and
a storage device in which a program is stored,
wherein the method according to any one of claims 1 to 6 is performed by the program executed by the processor.

10. A consensus diagnosis system comprising:
a processor; and
a storage device in which a program executed by the processor is stored,
wherein the program
checks N individual diagnosis results of given bio data, which are output by respective N (N is an integer equal to or greater than 2) diagnosis systems, wherein each of the N diagnosis systems is a system trained using learning data annotated by a different annotation subject, and
outputs a consensus diagnosis result of the bio data based on the checked individual diagnosis results.

11. The consensus diagnosis system of claim 10, wherein the program
further checks a standard-individual diagnosis result of the bio data, which is output by a standard diagnosis system trained by gold standard learning data, and
outputs the consensus diagnosis result based on the individual diagnosis results and the standard-individual diagnosis result.

12. The consensus diagnosis system of claim 10, wherein:
the program outputs the consensus diagnosis result based on a weight assigned to each of the diagnosis systems, and
the weight is determined based on a number of times that the consensus diagnosis result and each of individual diagnosis results of the diagnosis systems are different.

13. The consensus diagnosis system of claim 12, wherein the weight is determined further based on a number or percentage of other diagnosis systems which output individual diagnosis results different from the consensus diagnosis result among all the individual diagnosis results, when the specific diagnosis system outputs the individual diagnosis result different from the consensus diagnosis result.

14. The consensus diagnosis system of claim 11, wherein:
the program outputs the consensus diagnosis result based on a weight assigned to each of the diagnosis systems and the standard diagnosis system, and
a higher weight is assigned to the standard diagnosis system than to the diagnosis systems.

15. The consensus diagnosis system of claim 10, wherein:
the consensus diagnosis result and an individual diagnosis result of a specific diagnosis system among the diagnosis systems are different with respect to specific bio data of the bio data used for the diagnosis of the consensus diagnosis system, and
the specific diagnosis system is retrained by learning data comprising the specific bio data annotated as the consensus diagnosis result.
